(19)

Europäisches Patentamt
European Patent Office
Office européen des brevets

(11) **EP 4 706 508 A1**

(12) **EUROPÄISCHE PATENTANMELDUNG**

(43) Veröffentlichungstag:
**11.03.2026 Patentblatt 2026/11**

(21) Anmeldenummer: **25195708.0**

(22) Anmeldetag: **13.08.2025**

(51) Internationale Patentklassifikation (IPC):
***A61B 5/00*** *(2006.01)*

(52) Gemeinsame Patentklassifikation (CPC):
**A61B 5/374; A61B 5/1103; A61B 5/4806**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH LA MA MD TN**

(30) Priorität: **03.09.2024 DE 102024208368**

(71) Anmelder: **SOMNOmedics AG**
**97236 Randersacker (DE)**

(72) Erfinder: **Küchler, Gert**
**97236 Randersacker (DE)**

(74) Vertreter: **Rau, Schneck & Hübner**
**Patentanwälte Rechtsanwälte PartGmbB**
**Königstraße 2**
**90402 Nürnberg (DE)**

(54) **VORRICHTUNG ZUR INSOMNIE-BESTIMMUNG**

(57)      Die Vorrichtung (1) dient zur Bestimmung einer bei einem Patienten (8) vorliegenden Insomnie. Sie hat eine Sensoreinheit (2) mindestens zur Erfassung eines EEG-Messsignals eines Gehirnstroms des Patienten (8) und eine Steuer- und Auswerteeinheit (3) zur Auswertung des erfassten EEG-Messsignals. Die Steuer- und Auswerteeinheit (3) ist dazu ausgelegt, eine objektive Erfassungsroutine durchzuführen, und dabei dem Patienten (8) für die folgende Erfassung des EEG-Messsignals eine Vorgabe zur Augenöffnung für die Dauer einer Augenöffnungszeitspanne und zur Augenschließung für die Dauer einer Augenschließungszeitspanne zu machen, und danach die Erfassung des EEG-Messsignals während mindestens einer Augenöffnungszeitspanne und während mindestens einer Augenschließungszeitspanne zu veranlassen. Die Steuer- und Auswerteeinheit (3) ist weiterhin dazu ausgelegt, eine Auswertung des so erfassten EEG-Messsignals durchzuführen, und dabei aus dem EEG-Messsignal mindestens ein erstes Teilmesssignal, das innerhalb der mindestens einen Augenöffnungszeitspanne liegt, und mindestens ein zweites Teilmesssignal, das innerhalb der mindestens einen Augenschließungszeitspanne liegt, zu extrahieren, und auf das Vorliegen von Insomnie zu entscheiden, wenn das innerhalb der mindestens einen Augenschließungszeitspanne liegende mindestens eine zweite Teilmesssignal einen geringeren Anteil an $\alpha$-Wellen aufweist als bei einem gesunden Menschen.

Fig. 1

EP 4 706 508 A1

**Beschreibung**

Der Inhalt der deutschen Patentanmeldung DE 10 2024 208 368.2 wird durch Bezugnahme hierin aufgenommen.

Die Erfindung betrifft eine Vorrichtung zur Bestimmung einer bei einem Patienten vorliegenden Insomnie.

Unter Insomnie sind Ein- und/oder Durchschlafstörungen zu verstehen. Es handelt sich um eine der am weitesten verbreiteten und oft unerkannten Schlafstörungen, an der bis zu 30 % der erwachsenen Bevölkerung leiden. Trotz der hohen Häufigkeit dieser Schlafstörung gibt es bislang kein geeignetes Screeningverfahren oder -gerät zur Ermittlung eines objektiven Parameters, anhand dessen das Vorliegen von Insomnie diagnostiziert werden kann. Auch anhand der sehr aufwändigen, teuren und nur in einem Schlaflabor durchzuführenden Polysomnografie ist bislang kein solcher objektiver Insomnie-Parameter zu bestimmen.

Stattdessen wird seit einiger Zeit eine Insomnie-Diagnose nur aufgrund der subjektiven Befindlichkeit einer betroffenen Person gestellt. Grundlage der Insomnie-Diagnose sind ausschließlich Schlaf-Fragebögen, die die betroffene Person nach der nächtlichen Schlafenszeit am nächsten Morgen ausfüllt. Die so mittels der Schlaf-Fragebögen erfassten Feststellungen sind sehr subjektiv und hängen zumindest teilweise auch von dem Verlauf und der Qualität des vorangegangenen Schlafs ab.

Aus der DE 20 2022 106 837 U1 ist eine Vorrichtung zur Schlafdiagnose bekannt, die sich zu einem Einsatz in der häuslichen Umgebung des Patienten eignet. Mittels vom Patienten selbst auf seiner Kopfhaut anzubringender Elektroden werden an dem Patienten elektrophysiologische Signale erfasst, welche an eine mehrteilige bzw. -komponentige Steuer-/Auswerteeinheit zur dortigen Auswertung übermittelt werden. Bei diesen elektrophysiologischen Signalen handelt es sich insbesondere um Elektroenzephalografie (EEG)-Signale zur Erfassung der elektrischen Aktivität des Gehirns, um Elektrookulographie (EOG)-Signale zur Erfassung der Augenbewegung und/oder um Elektromyographie (EMG)-Signale zur Erfassung von Muskelaktivität im Kopfbereich. Diese Vorrichtung dient zwar der Schlafdiagnose, eine Insomnie-Diagnose lässt sich damit aber nicht durchführen.

Die Aufgabe der Erfindung besteht darin, eine Vorrichtung der eingangs bezeichneten Art mit gegenüber dem Stand der Technik verbesserten Eigenschaften anzugeben.

Zur Lösung dieser Aufgabe wird eine Vorrichtung entsprechend den Merkmalen des Patentanspruchs 1 angegeben. Die erfindungsgemäße Vorrichtung weist auf eine Sensoreinheit mindestens zur Erfassung eines EEG-Messsignals eines Gehirnstroms des Patienten sowie eine Steuer- und Auswerteeinheit zur Auswertung des erfassten EEG-Messsignals. Die Steuer- und Auswerteeinheit ist dazu ausgelegt, eine objektive Erfassungsroutine durchzuführen, und dabei dem Patienten für die folgende Erfassung des EEG-Messsignals eine Vorgabe zur Augenöffnung für die Dauer einer Augenöffnungszeitspanne und zur Augenschließung für die Dauer einer Augenschließungszeitspanne zu machen, und danach die Erfassung des EEG-Messsignals während mindestens einer Augenöffnungszeitspanne und während mindestens einer Augenschließungszeitspanne zu veranlassen. Die Steuer- und Auswerteeinheit ist weiterhin dazu ausgelegt, eine Auswertung des so erfassten EEG-Messsignals durchzuführen, und dabei aus dem EEG-Messsignal mindestens ein erstes Teilmesssignal, das innerhalb der mindestens einen Augenöffnungszeitspanne liegt, und mindestens ein zweites Teilmesssignal, das innerhalb der mindestens einen Augenschließungszeitspanne liegt, zu extrahieren, und auf das Vorliegen von Insomnie zu entscheiden, wenn das innerhalb der mindestens einen Augenschließungszeitspanne liegende mindestens eine zweite Teilmesssignal einen geringeren Anteil an $\alpha$-Wellen aufweist als bei einem gesunden Menschen.

Die Kurzbezeichnung "EEG" steht hier für Elektroenzephalografie bzw. für Elektroenzephalogramm.

Während der objektiven Erfassungsroutine sind insbesondere mindestens eine Augenöffnungszeitspanne und mindestens eine Augenschließungszeitspanne vorgesehen. Die Augen des Patienten oder Benutzers der Vorrichtung zur Insomnie-Bestimmung sind während der Augenöffnungszeitspanne geöffnet und während der Augenschließungszeitspanne geschlossen.

Ein gesunder Mensch hat im Wachzustand bei geöffneten Augen normalerweise eine hohe Hirnaktivität bzw. eine hohe Neuronen-Aktivität, wodurch das erfasste EEG-Messsignal einen maßgeblichen Anteil sogenannter $\beta$-Wellen aufweist. Werden die optischen Reize z.B. durch Schließen der Augen entfernt, nimmt die Neuronen-Aktivität ab und es erfolgen synchronisierte Neuronen-Entladungen. Das erfasste EEG-Messsignal weist dann einen maßgeblichen Anteil sogenannter $\alpha$-Wellen auf, die gegenüber den $\beta$-Wellen eine reduzierte Frequenz haben. Die $\alpha$-Wellen treten hauptsächlich bei geschlossenen Augen und entspannter Wachheit auf und werden mit dem Öffnen der Augen durch $\beta$-Wellen ersetzt und umgekehrt. Diese Erscheinung ist als Berger-Effekt bekannt.

Es wurde erkannt, dass bei Insomnikern beim Schließen der Augen kein vergleichbarer Wechsel hin zu $\alpha$-Wellen wie bei gesunden Menschen als mögliches Korrelat einer verminderten Entspannungsfähigkeit stattfindet. Daher kann das Fehlen von $\alpha$-Wellen beim Schließen der Augen als Maß für die Fähigkeit zu entspannen herangezogen werden. Auf dieser Erkenntnis basiert bei der erfindungsgemäßen Vorrichtung die Entscheidung auf Vorliegen von Insomnie. Diese Entscheidung erfolgt vorzugsweise automatisiert und insbesondere auch objektiv anhand des im Rahmen der objektiven Erfassungsroutinen bei geöffneten und geschlossenen Augen erfassten und danach ausgewerteten EEG-Messsignals.

Eine der Schwierigkeiten der herkömmlichen Insomnie-Diagnose anhand der Schlaf-Fragebögen ist die gegenläufige Bewertung von Schlafdruck und aktueller Vigilanz (= Wachheit). Besteht ein hoher Schlafdruck und eine niedrige Vigilanz, wird es keine Einschlafschwierigkeiten geben. Sind sowohl der Schlafdruck als auch die Vigilanz hoch, kann es zu Einschlafstörungen kommen. Bei hohem Schlafdruck und nur erhöhter Vigilanz kann das Einschlafverhalten noch gut sein, sich nach der ersten Tiefschlaf-Phase bei dann verringertem Schlafdruck ein verschlechtertes Durchschlafverhalten einstellen. Dies alles kann beim Ausfüllen des Schlaf-Fragebogens am nächsten Morgen mit einfließen und eine dann auf dieser Grundlage getroffene Insomnie-Diagnose verfälschen.

Diese Schwierigkeit wird durch die Vorrichtung zur Insomnie-Bestimmung vermieden, da die Insomnie-Diagnose anhand des als objektives Kriterium erkannten Fehlens eines maßgeblichen Anteils an $\alpha$-Wellen im EEG-Messsignal bei geschlossenen Augen vorgenommen wird. Dieses objektive Kriterium weist eine sehr gute Korrelation zur subjektiven Insomnie-Befindlichkeit des Patienten gemäß der von ihm ausgefüllten Schlaf-Fragebögen auf. Insgesamt vereinfacht und verbessert die erfindungsgemäße Vorrichtung die Insomnie-Diagnose erheblich. Außerdem objektiviert sie sie.

Insbesondere kann die Vorrichtung zusätzlich zur Insomnie-Bestimmung weitere Funktionen aufweisen, wie z.B. eine vorteilhafterweise einfache und kostengünstige Erkennung oder Erfassung der Schlafstadien des dann insbesondere mittels der Vorrichtung während des Schlafs überwachten Patienten. Insofern kann die Vorrichtung in dieser günstigen Ausgestaltung ein Schlaf-Screening durchführen. Sie kann dann auch als eine Screening-Vorrichtung bezeichnet werden und ist in dieser günstigen Ausgestaltung insbesondere multifunktional.

Die Sensoreinheit ist insbesondere dazu ausgelegt, neben dem EEG-Messsignal zur Erfassung der elektrischen Aktivität des Gehirns weitere elektrophysiologische Messsignale zu erfassen, wie z.B. ein Elektrookulographie (EOG)-Messsignal zur Erfassung der Augenbewegung oder ein Elektromyographie (EMG)-Messsignal zur Erfassung von Muskelaktivität im Kopfbereich. Außerdem ist die Sensoreinheit vorzugsweise dazu ausgelegt, weitere Messsignale, wie z.B. ein Körperlage-Messsignal und ein akustisches Messsignal eines vom Patienten während des Schlafs verursachten Geräuschs, insbesondere eines Schnarch-Geräuschs, zu erfassen. Außerdem kann die Sensoreinheit auch dazu ausgelegt sein, zusätzlich mindestens ein vom Patienten unabhängiges Messsignal, wie z.B. ein Licht-Messsignal, insbesondere das des Umgebungslichts, zu erfassen. Diese weiteren Messsignale dienen dann insbesondere auch zur Erkennung verschiedener Schlafzustände oder Schlafstadien des Patienten.

Vorteilhafte Ausgestaltungen der Vorrichtung ergeben sich aus den Merkmalen der von Anspruch 1 abhängigen Ansprüche.

Günstig ist eine Ausgestaltung, bei der die Steuer- und Auswerteeinheit dazu ausgelegt ist, das mindestens eine zweite Teilmesssignal auf das Vorliegen von $\alpha$-Zeitabschnitten, in denen $\alpha$-Wellen hervortreten, zu prüfen, und für jeden erkannten $\alpha$-Zeitabschnitt eine $\alpha$-Zeitabschnittsdauer zu ermitteln, und auf das Vorliegen von Insomnie mittleren bis schweren Grades zu entscheiden, wenn die Summe aller $\alpha$-Zeitabschnittsdauern bezogen auf eine Gesamtzeitdauer des mindestens einen zweiten Teilmesssignals kleiner als 20 % ist. Vorzugsweise sind die $\alpha$-Wellen innerhalb der $\alpha$-Zeitabschnitte gegenüber anderen Wellenanteilen des mindestens einen zweiten Teilmesssignals dominant. Insbesondere treten die $\alpha$-Wellen gegenüber anderen Wellenanteilen des mindestens einen zweiten Teilmesssignals hervor, vorzugsweise markant.

Gemäß einer weiteren günstigen Ausgestaltung ist die Steuer- und Auswerteeinheit dazu ausgelegt, einen Vergleich zwischen einem ersten Frequenzgehalt des innerhalb der mindestens einen Augenöffnungszeitspanne liegenden mindestens einen ersten Teilmesssignals und einem zweiten Frequenzgehalt des innerhalb der mindestens einen Augenschließungszeitspanne liegenden mindestens einen zweiten Teilmesssignals durchzuführen, und für den Vergleich aus dem ersten Frequenzgehalt des innerhalb der mindestens einen Augenöffnungszeitspanne liegenden mindestens einen ersten Teilmesssignals eine erste $\alpha$-Kenngröße für ein $\alpha$-Frequenzintervall und eine erste $\beta$-Kenngröße für ein $\beta$-Frequenzintervall sowie ein erstes $\alpha/\beta$-Verhältnis als Quotient der ersten $\alpha$-Kenngröße zu der ersten $\beta$-Kenngröße zu bestimmen, aus dem zweiten Frequenzgehalt des innerhalb der mindestens einen Augenschließungszeitspanne liegenden mindestens einen zweiten Teilmesssignals eine zweite $\alpha$-Kenngröße für das $\alpha$-Frequenzintervall und eine zweite $\beta$-Kenngröße für das $\beta$-Frequenzintervall sowie ein zweites $\alpha/\beta$-Verhältnis als Quotient der zweiten $\alpha$-Kenngröße zu der zweiten $\beta$-Kenngröße zu bestimmen, und eine relative Änderung zwischen dem ersten und dem zweiten $\alpha/\beta$-Verhältnis zu bestimmen. Bei dieser günstigen Ausgestaltung werden sowohl der Einfluss der $\alpha$-Wellen (beschrieben durch die erste und die zweite $\alpha$-Kenngröße) also auch der Einfluss der $\beta$-Wellen (beschrieben durch die erste und die zweite $\beta$-Kenngröße) berücksichtigt. Die $\alpha$-Wellen liegen in dem $\alpha$-Frequenzintervall, das sich insbesondere über Frequenzen zwischen 8 Hz und 14 Hz, vorzugsweise zwischen 8 Hz und 13 Hz und bevorzugt zwischen 8 Hz und 12 Hz erstreckt. Die $\beta$-Wellen liegen in dem $\beta$-Frequenzintervall, das sich insbesondere über Frequenzen zwischen 14 Hz und 32 Hz, vorzugsweise zwischen 14 Hz und 30 Hz und bevorzugt zwischen 13 Hz und 30 Hz erstreckt. Die neuronale Aktivität ist bei jedem Menschen unterschiedlich und hängt insbesondere unter anderem vom Alter und Geschlecht ab. Um den Einfluss dieser individuellen Unterschiede auf die Insomnie-Bestimmung gering zu halten oder sogar komplett zu eliminieren, werden bei dieser günstigen Ausgestaltung die für die Insomnie-Bestimmung maßgeblichen $\alpha$-Aktivitäten auf die $\beta$-Aktivitäten bezogen. Damit ergibt sich eine die individuelle neuronale Aktivität des betreffenden Patienten berücksichtigende Normierung, wodurch die Genauigkeit der Ergebnisse der Insomnie-Bestimmung verbessert wird. Es wurde erkannt, dass die relative Änderung zwischen dem ersten $\alpha/\beta$-Verhältnis bei geöffneten Augen und dem zweiten $\alpha/\beta$-Verhältnis bei geschlossenen Augen überraschenderweise eine sehr gute Korrelation zur subjektiven Insomnie-Befindlichkeit des Patienten gemäß der von ihm ausgefüllten Schlaf-Fragebögen aufweist. Weiterhin wurde erkannt, dass bei gesunden Personen eine signifikante relative Änderung zwischen dem ersten $\alpha/\beta$-Verhältnis bei geöffneten Augen und dem zweiten $\alpha/\beta$-Verhältnis bei geschlossenen Augen besteht, wohingegen bei Insomnikern diese relative Änderung, wenn sie denn überhaupt auftritt, nur sehr niedrig ist.

Die Steuer- und Auswerteeinheit ist insbesondere dazu ausgelegt, das mindestens eine erste Teilmesssignal (für Augenöffnung) in den Frequenzbereich zu transformieren, z.B. mittels einer Fast Fourier-Transformation (FFT), und das dabei resultierende erste Frequenzsignal für die Ermittlung der ersten $\alpha$-Kenngröße innerhalb des $\alpha$-Frequenzintervalls sowie für die Ermittlung der ersten $\beta$-Kenngröße innerhalb des $\beta$-Frequenzintervalls weiter auszuwerten. Die zweite $\alpha$-Kenngröße und die zweite $\beta$-Kenngröße des mindestens einen zweiten Teilmesssignals (für Augenschließung) werden analog zur ersten $\alpha$-Kenngröße und ersten $\beta$-Kenngröße ermittelt. So ist die Steuer- und Auswerteeinheit insbesondere auch dazu ausgelegt, das mindestens eine zweite Teilmesssignal (für Augenschließung) in den Frequenzbereich zu transformieren, z.B. ebenfalls mittels einer Fast Fourier-Transformation (FFT), und das dabei resultierende zweite Frequenzsignal für die Ermittlung der zweiten $\alpha$-Kenngröße innerhalb des $\alpha$-Frequenzintervalls sowie für die Ermittlung der zweiten $\beta$-Kenngröße innerhalb des $\beta$-Frequenzintervalls weiter auszuwerten. Die weitere Auswertung erfolgt beispielsweise durch Bestimmung der unter der Kurve des ersten bzw. des zweiten Frequenzsignals innerhalb des $\alpha$-Frequenzintervalls und innerhalb des $\beta$-Frequenzintervalls gebildeten Flächeninhalte. Die $\alpha$- und $\beta$-Kenngrößen können aber auch auf eine andere geeignete Weise ermittelt werden, beispielsweise durch Bestimmung der Energiegehalte des mindestens einen ersten Teilmesssignals (für Augenöffnung) sowie des mindestens einen zweiten Teilmesssignals (für Augenschließung) jeweils innerhalb des $\alpha$-Frequenzintervalls und innerhalb des $\beta$-Frequenzintervalls, wobei eine zusätzliche Quadrierung des betreffenden Signals erfolgt.

Um den Einfluss zufälliger Schwankungen zu unterbinden, kann die Steuer- und Auswerteeinheit insbesondere dazu ausgelegt sein, eine Mittelung über mehrere Einzelmessungen oder Einzelauswertungen vorzunehmen.

Gemäß einer weiteren günstigen Ausgestaltung ist die Steuer- und Auswerteeinheit dazu ausgelegt, die relative Änderung zwischen dem ersten und dem zweiten $\alpha/\beta$-Verhältnis als eine auf das erste $\alpha/\beta$-Verhältnis bezogene Differenz zwischen dem ersten $\alpha/\beta$-Verhältnis als Minuend und dem zweiten $\alpha/\beta$-Verhältnis als Subtrahend zu bestimmen, und auf das Vorliegen von Insomnie mittleren bis schweren Grades zu entscheiden, wenn die so bestimmte relative Änderung zwischen dem ersten und dem zweiten $\alpha/\beta$-Verhältnis größer als ein Insomnie-Grenzwert ist. Der Insomnie-Grenzwert liegt insbesondere im Bereich zwischen -45 % und -15 %, vorzugsweise im Bereich zwischen -35 % und -17 %, höchst vorzugsweise im Bereich zwischen -25 % und -19 % und bevorzugt bei -20 %.

Gemäß einer weiteren günstigen Ausgestaltung ist die Steuer- und Auswerteeinheit dazu ausgelegt, einen Insomnie-Grad IG gemäß der Gleichung:

$$IG = e^{\left(\frac{\Delta\alpha\beta + c1}{c2}\right)} \tag{1}$$

mit

$$\Delta\alpha\beta = \frac{\left(\frac{\alpha 1}{\beta 1} - \frac{\alpha 2}{\beta 2}\right)}{\frac{\alpha 1}{\beta 1}} \tag{2}$$

zu bestimmen, wobei c1 eine erste Konstante, c2 eine zweite Konstante und $\Delta\alpha\beta$ die relative Änderung zwischen dem ersten und dem zweiten $\alpha/\beta$-Verhältnis bezeichnet. Weiterhin steht $\alpha 1$ für die erste $\alpha$-Kenngröße während der Augen-öffnungszeitspanne, $\beta 1$ für die erste $\beta$-Kenngröße während der Augenöffnungszeitspanne, $\alpha 2$ für die zweite $\alpha$-Kenngröße während der Augenschließungszeitspanne und $\beta 2$ für die zweite $\beta$-Kenngröße während der Augenschließungszeit-spanne. Gemäß einer besonders bevorzugten Ausgestaltung hat die erste Konstante c1 einen Wert von insbesondere 110,4 und die zweite Konstante c2 einen Wert von insbesondere 31,5. Der Insomnie-Grad IG lässt sich so sehr zuverlässig und genau bestimmen. Die Sensitivität dieser Bestimmung liegt insbesondere bei 0,93 und die Spezifität insbesondere bei 0,76.

Gemäß einer weiteren günstigen Ausgestaltung ist die Steuer- und Auswerteeinheit dazu ausgelegt, die objektive Erfassungsroutine vor einem Einschlafen des Patienten durchzuführen, insbesondere unmittelbar vor dem Einschlafen. Bevorzugt erfolgt die Durchführung der objektiven Erfassungsroutine vor der normalerweise üblichen oder vor der tatsächlichen Schlafenszeit, also z.B. vor dem abendlichen Zubettgehen. Dies ist günstig, da die Vigilanz kurz vor dem Schlafen oder auch noch zu Beginn des Schlafs unabhängig vom danach folgenden Schlafverlauf ist und deshalb zu diesem Zeitpunkt bessere Resultate bei der Insomnie-Diagnose erzielt werden können.

Gemäß einer weiteren günstigen Ausgestaltung weist die Vorrichtung mindestens eine portable Teilkomponente auf. Die portable Teilkomponente kann insbesondere ein mobiles Gerät, wie z.B. ein Smartphone, ein Tablet-Computer, ein Laptop-Computer oder ein Wearable, sein. Vorzugsweise ist die Vorrichtung als eine vom Patienten selbst, und insbesondere in seiner häuslichen Umgebung, bedienbare Vorrichtung ausgeführt. Die Vorrichtung ist vorzugsweise in der häuslichen Umgebung des Patienten einsetz- oder verwendbar. Dies erleichtert die Handhabung und führt zu sehr guten Ergebnissen, da der Patient für die Insomnie-Diagnose nicht seine gewohnte Schlafumgebung verlassen und sich beispielsweise in einem Schlaflabor einer Untersuchung unterziehen muss. Letzteres kann auch zu einer Ergebnisverfälschung führen, da sich das Schlafverhalten aufgrund der ungewohnten Umgebung verändern kann.

Gemäß einer weiteren günstigen Ausgestaltung ist die Steuer- und Auswerteeinheit dazu ausgelegt, vor der Extraktion des mindestens einen ersten Teilmesssignals und des mindestens einen zweiten Teilmesssignals eine Artefakt-Bereinigung des EEG-Messsignals durchzuführen. Dadurch verbessert sich die Genauigkeit der aus dem erfassten EEG-Messsignal abgeleiteten Größen und Parameter, insbesondere die der Insomnie-Diagnose.

Gemäß einer weiteren günstigen Ausgestaltung ist die Steuer- und Auswerteeinheit dazu ausgelegt, für die mindestens eine Augenöffnungszeitspanne und die mindestens eine Augenschließungszeitspanne jeweils eine Zeitdauer von mindestens 2 Sekunden vorzusehen. Eine Obergrenze dieser Zeitdauer liegt insbesondere im Bereich zwischen 7 Sekunden und 25 Sekunden, vorzugsweise zwischen 8 Sekunden und 20 Sekunden und bevorzugt bei 10 Sekunden. Die Untergrenze von 2 Sekunden gewährleistet, dass die aus dem EEG-Messsignal extrahierten ersten und zweiten Teilmesssignale lang und aussagekräftig genug für die sich anschließende Auswertung sind. Kürzere Zeitdauern sind außerdem auch wenig praktikabel, da der Patient oft nicht schnell genug reagiert. Die genannten Werte(-Bereiche) für die Obergrenze der Zeitdauer führen einerseits zu gut auswertbaren aussagekräftigen ersten und zweiten Teilmesssignalen, verlangen dem Patienten andererseits keine unzumutbar große Geduld beim Verharren in der geforderten Augenlid-Position (Augen auf oder Augen zu) ab.

Gemäß einer weiteren günstigen Ausgestaltung ist die Steuer- und Auswerteeinheit dazu ausgelegt, während der objektiven Erfassungsroutine mindestens zwei Augenöffnungszeitspannen und mindestens zwei Augenschließungszeitspannen vorzusehen, wobei sich die Augenöffnungszeitspannen und die Augenschließungszeitspannen jeweils abwechseln und insbesondere jeweils unmittelbar aufeinander folgen. Je mehr Augenöffnungszeitspannen und Augenschließungszeitspannen während der objektiven Erfassungsroutine vorgegeben werden, umso mehr oder besser auswertbare erste und zweite Teilmesssignale lassen sich aus dem erfassten EEG-Messsignal extrahieren. Beispielsweise können jeweils zwei oder drei Augenöffnungszeitspannen und Augenschließungszeitspannen vorgesehen sein. Bevorzugt ist eine Ausgestaltung mit drei Augenöffnungszeitspannen und zwei Augenschließungszeitspannen.

Gemäß einer weiteren günstigen Ausgestaltung ist die Steuer- und Auswerteeinheit dazu ausgelegt, dem Patienten während der Durchführung der objektiven Erfassungsroutine insbesondere optisch und/oder akustisch anzuzeigen, wann die mindestens eine Augenöffnungszeitspanne und die mindestens eine Augenschließungszeitspanne jeweils beginnt und endet. Die Anzeige kann insbesondere rein akustisch erfolgen oder auch gemischt optisch/akustisch. So lässt sich der Beginn der Augenschließungszeit-spanne optisch oder akustisch anzeigen. Auf das Ende der Augenschließungszeitspanne lässt sich dagegen sinnvollerweise nur durch eine akustische Anzeige, wie z.B. die Wiedergabe eines Ansagetextes oder auch nur eines Signaltons, hinweisen, da der Patient bei geschlossenen Augen eine optische Anzeige nicht erkennen würde. Eine Aufforderung zum Starten der Augenschließungszeitspanne kann mittels einer optischen Anzeige erfolgen, z.B. mittels eines Bildschirmtextes oder eines insbesondere bewegten oder kleiner werdenden Bildschirmsymbols. So kann ein auf einem Display dargestellter anfangs relativ großer Punkt kleiner werden und beim vollständigen Verschwinden das Startsignal zum Augenschließen geben. Eine derartige insbesondere optische und/oder akustische Anzeige von Start und Ende der jeweiligen Augenöffnungs- oder Augenschließungszeitspanne vereinfacht die Handhabung der Vorrichtung und verbessert außerdem die Erfassungsgenauigkeit. Außerdem ist es insbesondere auch möglich, anhand des Verlaufs eines EMG-Messsignals, einem anderen insbesondere ebenfalls erfassten elektrophysiologischen Messsignal, das ein Nachvollziehen von Muskelaktivität an den Augenlidern erlaubt, die Augenlid-Position (Augen auf oder Augen zu) zu ermitteln, oder zu kontrollieren, ob der Patient die Aufforderung zur Augenöffnung oder Augenschließung befolgt hat.

Gemäß einer weiteren günstigen Ausgestaltung ist die Vorrichtung mehrkomponentig ausgeführt. Insbesondere die Steuer- und Auswerteeinheit kann mehrteilig bzw. mehrkomponentig und/oder zumindest teilweise, vorzugsweise vollständig, als mobile oder tragbare Einheit ausgeführt sein. Sie kann als Teilkomponenten zumindest eine insbesondere tragbare Preprocessing-Einheit, die z.B. in der Nähe der zur Messsignal-Erfassung eingesetzten Elektroden, beispielsweise am Kopf des Patienten, platzierbar ist, und eine insbesondere tragbare und am Bett des Patienten platzierbare Recheneinheit, wie z.B. einen Tablet-Computer, ein Smartphone, einen Laptop-Computer oder ein Wearable, haben. Zwischen den einzelnen Teilkomponenten der Steuer- und Auswerteeinheit besteht eine Daten- oder Kommunikationsverbindung, insbesondere eine Funk-Daten- oder Funk-Kommunikationsverbindung, beispielsweise gemäß dem Bluetooth-Standard.

Die Aufteilung auf mehrere Teilkomponenten vereinfacht die Handhabung der Vorrichtung und verbessert die Flexibilität beim Einsatz der Vorrichtung.

Gemäß einer weiteren günstigen Ausgestaltung ist die Steuer- und Auswerteeinheit dazu ausgelegt, eine subjektive Erfassungsroutine durchzuführen, und dabei mit dem Patienten, insbesondere vor dem Einschlafen am Abend oder nach dem Aufwachen am nächsten Morgen, eine Befragung zu seiner subjektiven Schlafwahrnehmung vorzunehmen und insbesondere anhand der Befragungsergebnisse eine weitere Insomnie-Bestimmung vorzunehmen. Insbesondere ist die Steuer- und Auswerteeinheit dazu ausgelegt, die Ergebnisse der objektiven Erfassungsroutine und die Ergebnisse der subjektiven Erfassungsroutine miteinander zu vergleichen oder die Ergebnisse der objektiven Erfassungsroutine anhand der Ergebnisse der subjektiven Erfassungsroutine zu verfeinern. Dies erhöht die Genauigkeit der Insomnie-Bestimmung.

Weitere Merkmale, Vorteile und Einzelheiten der Erfindung ergeben sich aus der nachfolgenden Beschreibung von Ausführungsbeispielen anhand der Zeichnung. Es zeigt:

Fig. 1    ein Blockschaltbild eines Ausführungsbeispiels einer Insomnie-Bestimmungs-Vorrichtung mit einer Sensoreinheit und einer Steuer- und Auswerteeinheit,

Fig. 2    eine Teildarstellung eines Ausführungsbeispiels einer Insomnie-Bestimmungs-Vorrichtung gemäß Fig. 1 mit am Kopf eines Patienten zur Erfassung elektrophysiologischer Messsignale angebrachten Messelektroden als Teil der Sensoreinheit, und einer Vorprozessierungseinheit als Teil der Steuer- und Auswerteeinheit,

Fig. 3    ein Zeitverlaufsdiagramm eines innerhalb einer Augenöffnungszeitspanne liegenden ersten Teilmesssignals eines an einer gesunden Person erfassten EEG-Messsignals,

Fig. 4    ein Frequenzgangdiagramm des in den Frequenzbereich transformierten ersten Teilmesssignals gemäß Fig. 3,

Fig. 5    ein Zeitverlaufsdiagramm eines innerhalb einer Augenschließungszeitspanne liegenden zweiten Teilmesssignals eines an einer gesunden Person erfassten EEG-Messsignals,

Fig. 6    ein Frequenzgangdiagramm des in den Frequenzbereich transformierten zweiten Teilmesssignals gemäß Fig. 5,

Fig. 7    ein Zeitverlaufsdiagramm eines innerhalb einer Augenöffnungszeitspanne liegenden ersten Teilmesssignals eines an einem Insomniker erfassten EEG-Messsignals,

Fig. 8    ein Frequenzgangdiagramm des in den Frequenzbereich transformierten ersten Teilmesssignals gemäß Fig. 7,

Fig. 9    ein Zeitverlaufsdiagramm eines innerhalb einer Augenschließungszeitspanne liegenden zweiten Teilmesssignals eines an einem Insomniker erfassten EEG-Messsignals,

Fig. 10   ein Frequenzgangdiagramm des in den Frequenzbereich transformierten zweiten Teilmesssignals gemäß Fig. 9,

Fig. 11   ein Diagramm mit über dem Insomnie-Grad aufgetragener relativen Änderung zwischen dem ersten $\alpha/\beta$-Verhältnis bei geöffneten Augen und dem zweiten $\alpha/\beta$-Verhältnis bei geschlossenen Augen,

Fig. 12   Zeitverlaufsdiagramme eines innerhalb einer Augenschließungszeitspanne liegenden zweiten Teilmesssignals eines an einer gesunden Person erfassten EEG-Messsignals sowie von zwei EOG-Messsignalen, und

Fig. 13   Zeitverlaufsdiagramme eines innerhalb einer Augenschließungszeitspanne liegenden zweiten Teilmesssignals eines an einem Insomniker erfassten EEG-Messsignals sowie von zwei EOG-Messsignalen.

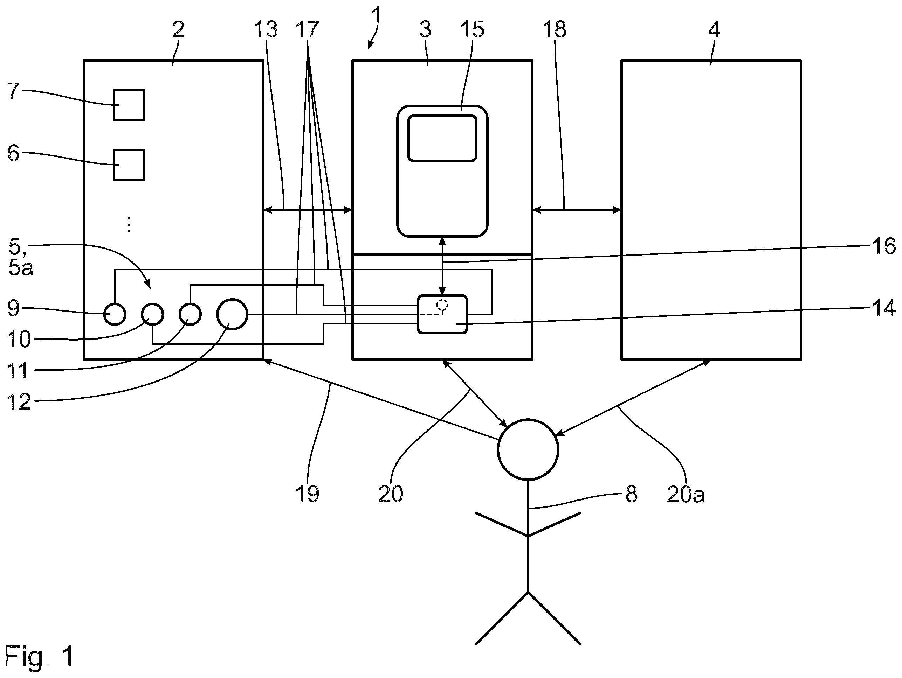

Einander entsprechende Teile sind in den Fig. 1 bis 13 mit denselben Bezugszeichen versehen. Auch Einzelheiten der im Folgenden näher erläuterten Ausführungsbeispiele können für sich genommen eine Erfindung darstellen oder Teil eines Erfindungsgegenstands sein.

In Fig. 1 ist ein Ausführungsbeispiel einer Insomnie-Bestimmungs-Vorrichtung 1 in einer Blockschaltbilddarstellung gezeigt. Die Insomnie-Bestimmungs-Vorrichtung 1 weist eine Sensoreinheit 2, eine Steuer- und Auswerteeinheit 3 sowie eine optionale Ferneinheit 4 auf.

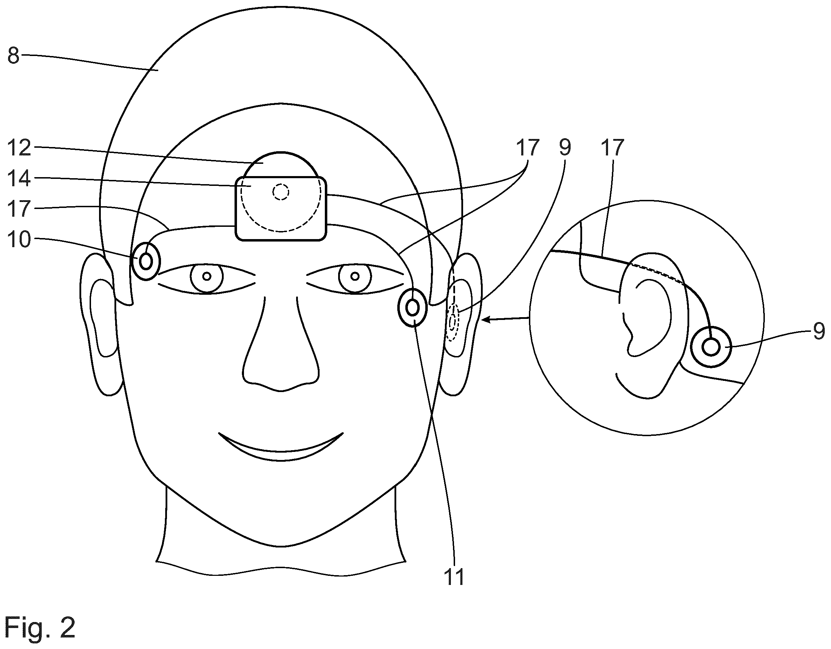

Die Sensoreinheit 2 enthält mehrere Sensoren 5, 6 und 7, die zur Erfassung von Messsignalen bestimmt sind, welche in Zusammenhang mit dem Schlafverhalten eines Patienten 8 stehen. Die Sensoren 5 sind zur Erfassung elektrophysiologischer Messsignale ausgelegt und enthalten dazu mehrere Messelektroden 9, 10, 11 und 12, die auf der Kopfhaut des Patienten 8 zu platzieren sind. Gemäß der Darstellung von Fig. 2 ist die Messelektrode 9, die insbesondere als eine Masseelektrode ausgeführt ist, hinter einem Ohr des Patienten 8 zu platzieren. Die Messelektrode 10 ist rechts neben den Augen und die Messelektrode 11 gegenüberliegend links neben den Augen zu platzieren. Die Messelektrode 12 ist insbesondere mittig auf der Stirn zu platzieren. Die Messelektroden 9 bis 12 erfassen jeweils elektrische Potentiale. Die Potentialunterschiede zwischen jeweils zwei der Messelektroden 9 bis 12 werden als elektrophysiologische Messsignale erfasst. So liefert die Potentialdifferenz zwischen der mittigen Messelektrode 12 und der Masseelektrode 9 ein EEG (Elektroenzephalographie)-Messsignal zur Erfassung der elektrischen Aktivität des Gehirns, also von Hirnströmen, die Potentialdifferenz zwischen der linken Messelektrode 11 und der mittigen Messelektrode 12 ein EOG (Elektrookulographie)-Messsignal für die Bewegung des linken Auges, die Potentialdifferenz zwischen der rechten Messelektrode 10 und der mittigen Messelektrode 12 ein weiteres EOG-Messsignal für die Bewegung des rechten Auges und die Potentialdifferenz zwischen der rechten Messelektrode 10 und der linken Messelektrode 11 ein EMG(Elektromyographie)-Messsignal zur Erfassung von Muskelaktivitäten in diesem Kopfbereich. Das Paar der mittigen Messelektrode 12 und der Masseelektrode 9 stellt einen EEG-Sensor 5a dar, wobei zumindest die mittige Messelektrode 12 zusätzlich für andere sensorische Erfassungen verwendet werden kann, insbesondere zur Erfassung von EOG-Messsignalen. Darüber hinaus sind in der Sensoreinheit 2 weitere Sensoren vorhanden, nämlich zumindest die Sensoren 6 und 7. Der Sensor 6 ist als Lagesensor ausgeführt und dient zur Erfassung der Kopfposition und/oder von Kopfbewegungen des Patienten 8. Der Sensor 7 ist ein akustischer Sensor und dient zur Erfassung von Schnarchgeräuschen des Patienten 8. Weitere Sensoren können vorhanden sein, beispielsweise ein Lichtsensor zur Erfassung von Umgebungslicht. Die von den Sensoren 5 bis 7 erfassten Messsignale werden an die Steuer- und Auswerteeinheit 3 übertragen. Hierzu steht eine unidirektionale oder bidirektionale Kommunikationsverbindung 13 zur Verfügung, die entweder als eine drahtgebundene Kommunikationsverbindung 17 oder als eine Funk-Kommunikationsverbindung, beispielsweise nach dem Bluetooth-Standard, ausgeführt ist. Beim gezeigten Ausführungsbeispiel erfolgt die Kommunikation zwischen den Sensoren 5 bzw. deren Messelektroden 9 bis 12 und der Steuer- und Auswerteeinheit 3 drahtgebunden und die Kommunikation zwischen den weiteren Sensoren 6 und 7 und der Steuer- und Auswerteeinheit 3 per Funk. Andere Aufteilungen oder Varianten sind aber grundsätzlich ebenfalls möglich.

Die Steuer- und Auswerteeinheit 3 ist bei dem gezeigten Ausführungsbeispiel mehrteilig oder mehrkomponentig ausgeführt. Sie umfasst eine Vorprozessierungseinheit 14 (Preprocessing-Einheit) sowie eine mobile Recheneinheit 15 in Form eines Tablet-Computers. Zwischen der Vorprozessierungseinheit 14 und der mobilen Recheneinheit 15 besteht ebenfalls eine Kommunikationsverbindung 16, die bei dem gezeigten Ausführungsbeispiel vorzugsweise als Funk-Kommunikationsverbindung nach dem Bluetooth-Standard ausgeführt ist. Sie ist insbesondere bidirektional. Gemäß der Darstellung von Fig. 2 ist die Vorprozessierungseinheit 14 auf die mittige Messelektrode 12 ausgesetzt, wobei zugleich eine elektrische Kommunikationsverbindung zwischen diesen beiden Komponenten hergestellt wird. Zu den übrigen Messelektroden 9, 10 und 11 besteht jeweils eine drahtgebundene Kommunikationsverbindung 17.

Die Ferneinheit 4 ist als eine fern vom Patienten 8 befindliche stationäre Recheneinheit ausgeführt, beispielsweise als ein Server oder als ein Cloud-Computer. Die Ferneinheit 4 kann auch einen Datenspeicher mit großem Speichervolumen, wie z.B. einen Cloud-Speicher, haben. Zum Datenaustausch besteht zwischen der Ferneinheit 4 und der Steuer- und Auswerteeinheit 3 eine Internet-Kommunikationsverbindung 18. Die von der Sensoreinheit 2 und/oder der Steuer- und Auswerteinheit 3 ermittelten Daten können an die Ferneinheit 4 übertragen werden, um dort beispielsweise weitere Auswertungen vorzunehmen oder einer medizinischen Fachkraft für deren Beurteilung Zugriff zu bieten.

Der Patient 8 steht mit den verschiedenen Einheiten der Insomnie-Bestimmungs-Vorrichtung 1 in Wechselwirkung. Bei der Sensoreinheit 2 bezieht sich diese Wechselwirkung auf physikalische Größen, die von den Sensoren 5 bis 7 der Sensoreinheit 2 am Patienten erfasst werden können. Diese Wechselwirkung 19 ist somit vom Patienten 8 zur Sensoreinheit 2 gerichtet. Sie ist insbesondere unidirektional. Die Wechselwirkung 20 zwischen dem Patienten 8 und der Steuer- und Auswerteeinheit 3 ist dagegen wie die Wechselwirkung 20a zwischen dem Patienten 8 und der Ferneinheit 4 bidirektional ausgestaltet. Der Patient 8 kann Informationen von der Steuer- und Auswerteeinheit 3 erhalten, beispielsweise visuell oder akustisch. Umgekehrt kann der Patient 8 Eingaben an die Steuer- und Auswerteeinheit 3 richten, beispielsweise im Rahmen einer anfänglich durchzuführenden Kalibrierungsroutine, einer objektiven Erfassungsroutine und/oder einer subjektiven Erfassungsroutine mit einer Befragung des Patienten 8.

Während der Kalibrierungsroutine werden insbesondere sich aus den aktuell beim Patienten 8 herrschenden Verhältnissen ergebende Grundeinstellungen der Insomnie-Bestimmungs-Vorrichtung 1 ermittelt. Weiterhin werden die Position und der Sitz der vom Patienten 8 angelegten Messelektroden 9 bis 12 kontrolliert, insbesondere mittels einer Fotografie.

Die Insomnie-Bestimmungs-Vorrichtung 1 zeichnet sich dadurch aus, dass sie vom Patienten 8 selbst bedient und vor allem auch in seiner häuslichen Umgebung angewendet werden kann.

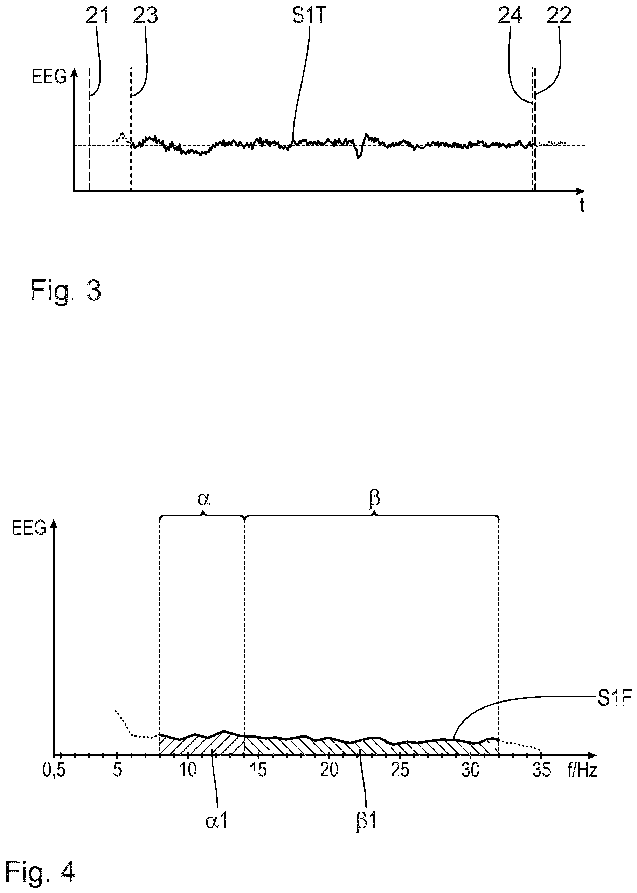

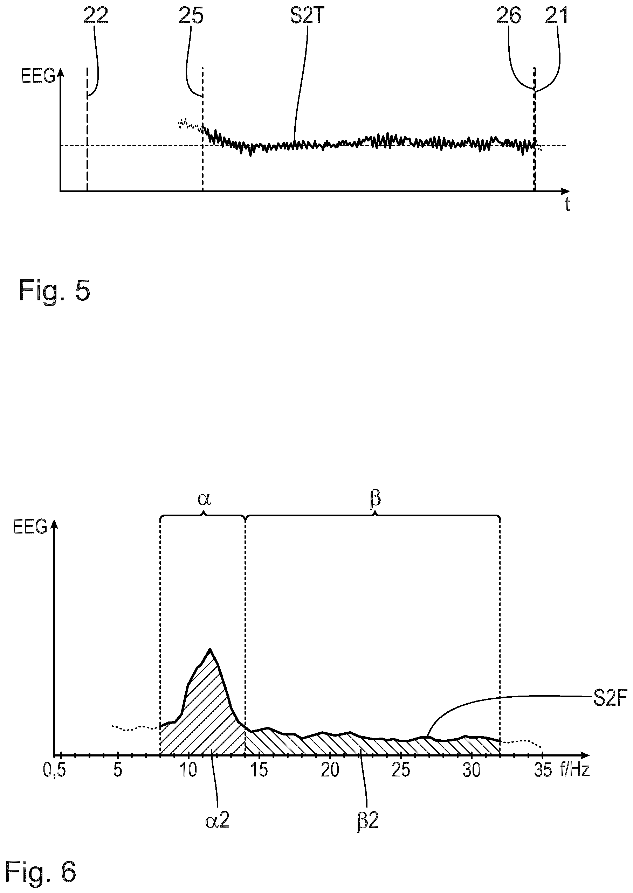

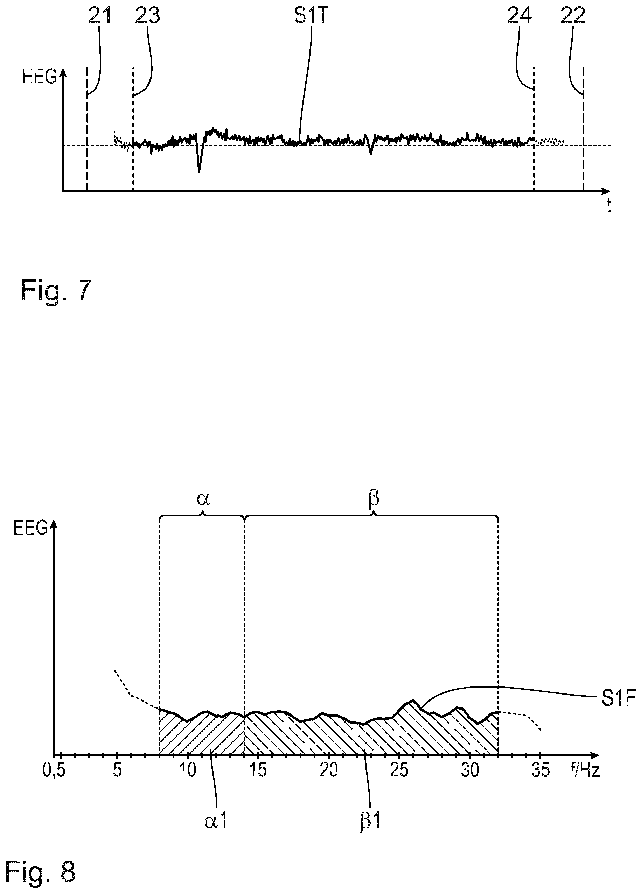

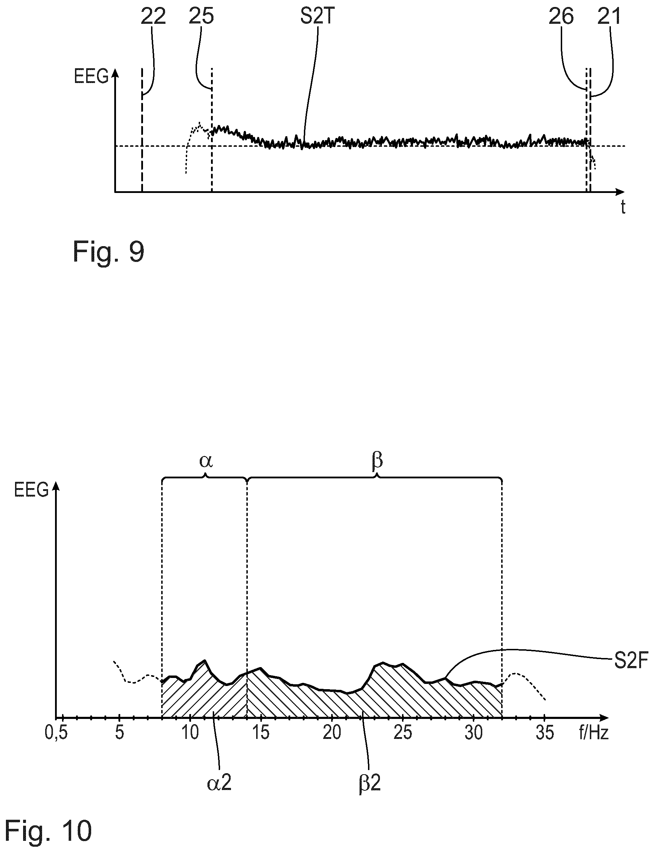

Die Steuer- und Auswerteeinheit 3 ist dazu ausgelegt, eine objektive Erfassungsroutine durchzuführen, bei der dem Patienten 8 Vorgaben zur Augenöffnung und Augenschließung gemacht werden, um bei der Erfassung des EEG-Messsignals Zeitspannen mit abzudecken, bei denen die Augen des Patienten 8 zum einen geöffnet (= Augenöffnungszeitspannen) und zum anderen geschlossen (= Augenschließungszeitspannen) sind. Bei der nachfolgenden Auswertung des so erfassten EEG-Messsignals werden Teilmesssignale extrahiert, die gerade innerhalb solcher spezieller Zeitspannen liegen. Erste Teilmesssignale S1T liegen jeweils innerhalb einer Augenöffnungszeitspanne, zweite Teilmesssignale S2T jeweils innerhalb einer Augenschließungszeitspanne. Erste Teilmesssignale S1T sind im Diagramm gemäß Fig. 3 (für einen gesunden Menschen) sowie in Fig. 7 (für eine an Insomnie leidende Person (= Insomniker)) beispielhaft gezeigt. Analog sind zweite Teilmesssignale S2T beispielhaft in Fig. 5 (für eine gesunde Person) sowie in Fig. 9 (für einen Insomniker) gezeigt. Bei diesen Diagramen handelt es sich jeweils um über der Zeit t dargestellte Verläufe. In den Zeitverläufen gemäß Fig. 3, 5, 7 und 9 sind die Zeitpunkte 21, zu denen von der Steuer- und Auswerteeinheit 3 eine Aufforderung zur Augenöffnung ergangen ist, sowie die Zeitpunkte 22, zu denen von der Steuer- und Auswerteeinheit 3 eine Aufforderung zur Augenschließung ergangen ist, mit eingetragen. Der Beginn des extrahierten ersten Teilmesssignals S1T ist mit 23, sein Ende mit 24 und der Beginn des extrahierten zweiten Teilmesssignals S2T mit 25 und dessen Ende mit 26 gekennzeichnet. Die Dauer der ersten und zweiten Teilmesssignale S1T, S2T ist jeweils kürzer als die von der Steuer- und Auswerteeinheit 3 vorgegebenen Augenöffnungs- und Augenschließungszeitspannen, da beim Wechsel der Augenlid-Position Übergangseffekte im erfassten EEG-Messsignal auftreten, die die weitere Auswertung verfälschen würden und deshalb nicht mit berücksichtigt werden. Die Steuer- und Auswerteeinheit 3 ist außerdem zur Beseitigung auch anderer Artefakte ausgelegt, um die Qualität der nachfolgenden Auswertung zu steigern. Diese Artefakt-Bereinigung kann insbesondere auch schon vor der Extraktion der ersten und zweiten Teilmesssignale S1T, S2T erfolgen. Die zeitliche Dauer der extrahierten ersten und zweiten Teilmesssignale S1T, S2T beträgt mindestens 2 Sekunden und liegt typischerweise im Bereich von 5 bis 10 Sekunden.

Die extrahierten ersten und zweiten Teilmesssignale S1T, S2T werden mittels einer Fast Fourier-Transformation (FFT) in den Frequenzbereich transformiert, wodurch die jeweils zugehörigen transformierten ersten Frequenzsignale S1F (für Augenöffnung) und zweiten Frequenzsignale S2F (für Augenschließung) resultieren. Die so gebildeten ersten Frequenzsignale S1F sind in den Frequenzdiagrammen gemäß Fig. 4 und 8, die zweiten Frequenzsignale S2F in den Frequenzdiagrammen gemäß Fig. 6 und 10 dargestellt. Die ersten und zweiten Frequenzsignale S1F, S2F sind dabei jeweils über der Frequenz f aufgetragen. Von diesen Frequenzgängen interessieren hier jeweils nur das $\alpha$-Frequenzin-tervall zwischen 8 Hz und 14 Hz sowie das sich anschließende $\beta$-Frequenzintervall zwischen 14 Hz und 32 Hz. Die $\alpha$- und $\beta$-Frequenzintervalle sind in Fig. 4, 6, 8 und 10 jeweils kenntlich gemacht. Mit dem EEG-Messsignal wird die Hirnstrom-Aktivität des Patienten 8 erfasst. Diese Aktivität verändert sich. Das EEG-Messsignal kann insofern auch verschiedene Anteile mit jeweils unterschiedlichem Frequenzgehalt beinhalten. Insbesondere kann es sogenannte $\alpha$-Wellen auf-weisen, deren Frequenzen in dem genannten $\alpha$-Frequenzintervall liegen und die üblicherweise dann auftreten, wenn der Patient 8 wach ist und die Augen geschlossen hat. Außerdem kann das EEG-Messsignal auch sogenannte $\beta$-Wellen enthalten, deren Frequenzen in dem ebenfalls bereits genannten $\beta$-Frequenzintervall liegen und die üblicherweise dann auftreten, wenn Patient 8 wach ist und die Augen geöffnet hat. Verändert der Patient 8 die Augenlid-Position, ist dies bei gesunden Personen auch im Frequenzverhalten abzulesen. Maßgeblich ist hier der Vergleich des in Fig. 4 wiedergegebenen ersten Frequenzsignals S1F mit dem im in Fig. 6 wiedergegebenen zweiten Frequenzsignal S2F. Es fällt auf, dass der Frequenzgehalt im $\alpha$-Frequenzintervall beim zweiten Frequenzsignal S2F (Augenschließung) gemäß Fig. 6 höher liegt als bei dem ersten Frequenzsignal S1F (Augenöffnung) gemäß Fig. 4. Dieser Unterschied ist typisch und wird als Berger-Effekt bezeichnet. Es wurde erkannt, dass dieser Wechsel im Frequenzgehalt innerhalb des $\alpha$-Frequenzin-tervalls bei einer Veränderung zwischen Augenöffnung und Augenschließung bei Insomnikern nicht in vergleichbarer Weise gegeben ist wie bei gesunden Menschen. Dies lässt sich auch anhand der einschlägigen Diagramme gemäß Fig. 8 und 10 ablesen. Bei Insomnikern kommt es bei Augenschließung nicht zu einer Erhöhung des Frequenzgehalts im $\alpha$-Frequenzintervall wie bei gesunden Menschen. Diese Erkenntnis wird in der Insomnie-Bestimmungs-Vorrichtung 1 für eine objektive Bestimmung von Insomnie bzw. eines Insomnie-Grades genutzt.

So ist die Steuer- und Auswerteeinheit 3 dazu ausgelegt, erste $\alpha$-Kenngrößen $\alpha1$ und erste $\beta$-Kenngrößen $\beta1$ aus dem ersten Frequenzsignal S1F zu ermitteln, nämlich insbesondere durch Bestimmung des Flächeninhalts unter der Kurve des Frequenzsignals S1F innerhalb des $\alpha$-Frequenzintervalls und innerhalb des $\beta$-Frequenzintervalls. Die für die erste $\alpha$-Kenngröße $\alpha1$ und die erste $\beta$-Kenngröße $\beta1$ maßgeblichen Flächeninhalte sind in den Diagrammen gemäß Fig. 4 und 8 durch unterschiedliche Schraffuren kenntlich gemacht. Dementsprechend werden eine zweite $\alpha$-Kenngröße $\alpha2$ und eine zweite $\beta$-Kenngröße $\beta2$ anhand des zweiten Frequenzsignals S2F ermittelt. Auch hier werden Flächeninhalte unterhalb der Kurven des zweiten Frequenzsignals S2F ermittelt. Die entsprechenden Flächen sind in den hierfür maßgeblichen Fig. 6 und 10 wiederum durch Schraffuren kenntlich gemacht. Die Steuer- und Auswerteeinheit bildet aus diesen $\alpha$- und $\beta$-Kenngrößen $\alpha1$, $\alpha2$, $\beta1$, $\beta2$ ein erstes $\alpha/\beta$-Verhältnis als Quotient der ersten $\alpha$-Kenngröße $\alpha1$ zu der ersten $\beta$-Kenngröße $\beta1$ sowie ein zweites $\alpha/\beta$-Verhältnis als Quotient der zweiten $\alpha$-Kenngröße $\alpha2$ zu der zweiten $\beta$-Kenngröße $\beta2$. Daraus wird gemäß der vorstehenden Gleichung (2) die relative Änderung $\Delta\alpha\beta$ zwischen dem ersten und dem zweiten $\alpha/\beta$-Verhältnis gebildet.

Bei gesunden Personen ergibt sich eine signifikante Änderung zwischen dem ersten $\alpha/\beta$-Verhältnis bei geö-

ffneten Augen und dem zweiten $\alpha/\beta$-Verhältnis bei geschlossenen Augen, wohingegen diese relative Veränderung bei Insomnikern entweder gar nicht auftritt oder nur sehr gering ausfällt. Die relative Änderung $\Delta\alpha\beta$ zwischen dem ersten und dem zweiten $\alpha/\beta$-Verhältnis kann somit als Maß für eine beim Patienten 8 vorliegende Insomnie herangezogen werden.

Hierbei handelt es sich um eine objektive Bestimmung der Insomnie, was gegenüber der bislang ausschließlich praktizierten Fragebogen-Methode, die sehr stark durch subjektive Wahrnehmungen des Patienten 8 bestimmt ist, Vorteile bietet. Ein Insomnie-Grad IG des Patienten 8 lässt sich aus der messtechnisch und durch Folgeauswertungen ermittelten relativen Änderungen $\Delta\alpha\beta$ zwischen dem ersten $\alpha/\beta$-Verhältnis bei Augenöffnung und dem zweiten $\alpha/\beta$-Verhältnis bei Augenschließung anhand der vorstehenden Gleichung (1) bestimmen. Die erste Konstante c1 beträgt dabei insbesondere 110,4 und die zweite Konstante c2 insbesondere 31,5 an. Der funktionelle Zusammenhang gemäß Gleichung (1) oder deren inverse Formulierung gemäß der folgenden Gleichung (3):

$$\Delta\alpha\beta = [c2 * \ln(IG)] - c1 \qquad (3)$$

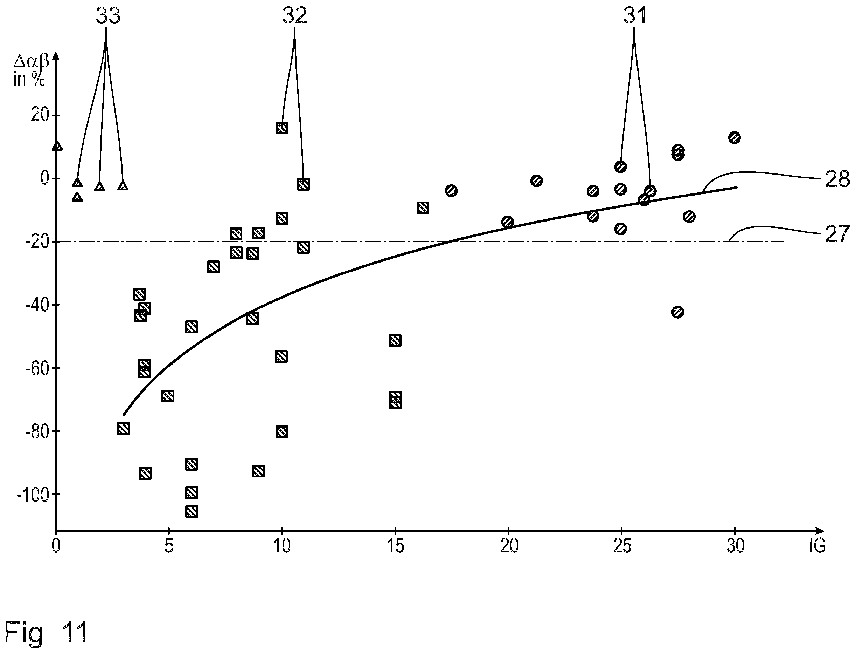

ist in dem Diagramm gemäß Fig. **11** durch die mit durchgezogener Linienführung wiedergegebene Kurve 28 dargestellt. Durch diesen Funktionszusammenhang ist die Insomnie-Bestimmungs-Vorrichtung 1 in der Lage, für den Patienten 8, der die objektive Erfassungsroutine durchlaufen hat, einen Insomnie-Grad IG anzugeben. Bevorzugt wird diese objektive Erfassungsroutine vor dem Einschlafen durchgeführt, was ebenfalls Vorteile gegenüber der Fragebogen-Methode bietet, die erst nach der Schlafphase am darauffolgenden Morgen unter den möglicherweise verfälschenden Eindrücken der vorangegangenen Schlafphase durchgeführt wird.

Alternativ oder ergänzend zu der funktionalen Bestimmung eines Insomnie-Grads IG kann in der Steuer- und Auswerteeinheit 3 auch nur überprüft werden, ob die ermittelte relative Änderung $\Delta\alpha\beta$ zwischen dem ersten und dem zweiten $\alpha/\beta$-Verhältnis über einen Insomnie-Grenzwert 27 (im Diagramm von Fig. 11 als strichpunktierte horizontale Linie wiedergegeben) liegt. Der Insomnie-Grenzwert 27 beträgt bei dem gezeigten Ausführungsbeispiel -20 %. Liegt die relative Änderung $\Delta\alpha\beta$ darüber, entscheidet die Steuer- und Auswerteeinheit 3 auf Vorliegen einer mittleren bis schweren Insomnie.

Im Diagramm von Fig. 11 sind außerdem auch die Ergebnisse einer Testreihe mit eingetragen. Die Test-Personen haben dabei sowohl die Fragebogen-Methode zur Insomnie-Diagnose als auch die objektive Erfassungsroutine der Insomnie-Bestimmungs-Vorrichtung 1 durchlaufen, so dass für sie jeweils ein gemäß der Fragebogen-Methode ermittelter Insomnie-Grad IG sowie eine gemäß der Insomnie-Bestimmungs-Vorrichtung 1 ermittelte relative Änderung $\Delta\alpha\beta$ zwischen dem ersten und dem zweiten $\alpha/\beta$-Verhältnis vorliegen. Beide Ergebnisse sind einander zugeordnet als Resultat der betreffenden Test-Person in das Diagramm gemäß Fig. 11 eingetragen. Die schraffierten Kreise 31 beziehen sich auf die Ergebnisse von Insomnikern, die schraffierten Quadrate 32 auf die Ergebnisse von gesunden Personen und die schraffierten Dreiecke 33 auf die Ergebnisse von Test-Personen, bei denen Messfehler erkannt worden sind und die deshalb als Ausreißer zu werten sind. Diese Ergebnisse der Test-Personen sind einem logarithmischen Fit unterzogen worden, wodurch sich der funktionale Zusammenhang gemäß den Gleichungen (1) bzw. (3), in Fig. 11 dargestellt mittels der Kurve 28, ergibt. Es liegt eine sehr hohe Sensitivität von 0,93 und eine ebenfalls sehr hohe Spezifität von 0,76 vor.

Die Steuer- und Auswerteeinheit 3 kann insbesondere dazu ausgelegt sein, zusätzlich zu der erläuterten objektiven Erfassungsroutine auch eine subjektive Erfassungsroutine basierend auf der Fragebogen-Methode durchzuführen. Die Ergebnisse dieser subjektiven Erfassungsroutine können dann dazu herangezogen werden, die Ergebnisse der objektiven Erfassungsroutine zu verifizieren und/oder zu verbessern.

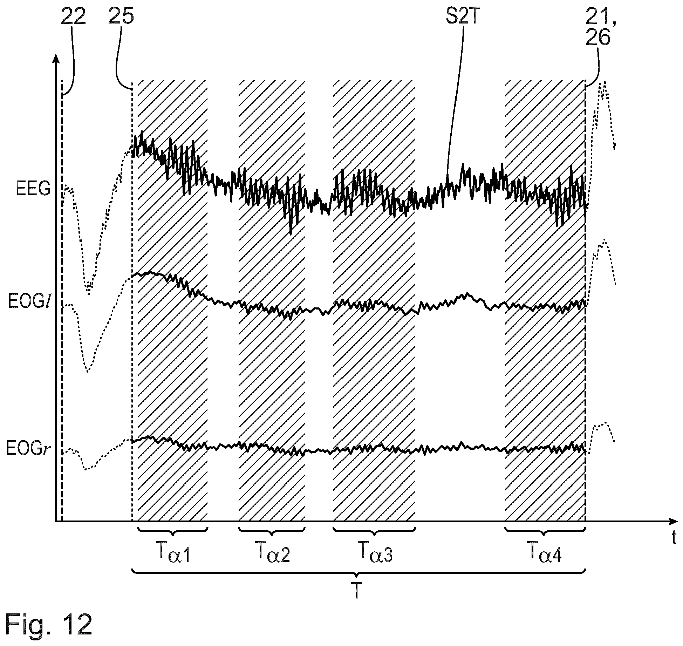

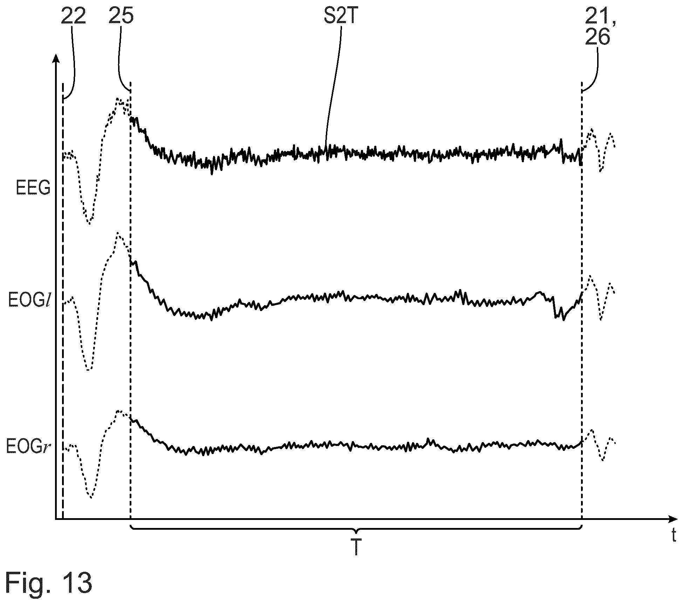

Im Folgenden wird anhand von Fig. 12 und 13 eine alternative Methode zur objektiven Insomnie-Bestimmung beschrieben, die ergänzend oder alternativ in der Insomnie-Bestimmungs-Vorrichtung 1 umgesetzt sein kann.

In Fig. 12 und 13 sind jeweils drei Zeitverlaufsdiagramme eines EEG-Messsignals sowie eines EOG-Messsignales des linken Auges (in Fig. 12 und 13 bezeichnet mit EOGl) und eines EOG-Messsignales des rechten Auges (in Fig. 12 und 13 bezeichnet mit EOGr) dargestellt. Die Messsignale gemäß Fig. 12 stammen von einer gesunden Person, die gemäß Fig. 13 von einem Insomniker. Gezeigt ist jeweils die Augenschließungszeitspanne, während der bei der gesunden Person ein erkennbarer und maßgeblicher Anteil an $\alpha$-Wellen vorliegt, wohingegen bei dem Insomniker kein vergleichbarer Anteil an $\alpha$-Wellen gegeben ist.

Von den EEG-Messsignalen gemäß Fig. 12 und 13 werden wiederum nur die um Artefakte bereinigten zweiten Teilmesssignale S2T näher untersucht. Die unberücksichtigten Artefakte treten vor allem (aber nicht nur) anfangs zwischen dem jeweiligen Zeitpunkt 22 mit der Aufforderung zur Augenschließung und dem jeweiligen Beginn 25 des weiter untersuchten zweiten Teilmesssignals S2T auf. In diese Phase fallen die anhand der EOG-Messsignale gemäß Fig. 12 und 13 ersichtlichen Bewegungen der Augenlider aufgrund der Aufforderung zur Augenschließung. Das Ende 26 des zweiten Teilmesssignals S2T und der Zeitpunkt 21 mit der Aufforderung zur Augenöffnung fallen bei den in Fig. 12 und 13 beispielhaft gezeigten Zeitverläufen zusammen.

Die Steuer- und Auswerteeinheit 3 ist dazu ausgelegt, die zweiten Teilmesssignale S2T daraufhin zu unter-

suchen, ob sie $\alpha$-Zeitabschnitte aufweisen, in denen $\alpha$-Wellen insbesondere gegenüber anderen Wellenanteilen des zweiten Teilmesssignals S2T insbesondere markant hervortreten und/oder insbesondere gegenüber anderen Wellenanteilen des zweiten Teilmesssignals S2T dominant sind. Dies erfolgt beispielsweise anhand von Mustererkennung. Das an der gesunden Person erfasste zweite Teilmesssignal S2T gemäß Fig. 12 hat 4 solcher $\alpha$-Zeitabschnitte mit $\alpha$-Zeitabschnittsdauern $T_{\alpha 1}$, $T_{\alpha 2}$, $T_{\alpha 3}$ und $T_{\alpha 4}$. Das am Insomniker erfasste zweite Teilmesssignal S2T gemäß Fig. 13 hat dagegen keinen $\alpha$-Zeitabschnitt. Beide Situationen werden von der Steuer- und Auswerteeinheit 3 erkannt und für die daraus abgeleitete Insomnie-Diagnose verwendet. Demnach wird auf Vorliegen einer Insomnie mittleren bis schweren Grades entschieden, wenn die Bedingung gemäß der folgenden Gleichung (4):

$$\frac{\sum_i T_{\alpha i}}{T} < 0{,}2 \qquad\qquad (4)$$

erfüllt ist, wobei T die Gesamtzeitdauer des jeweils untersuchten zweiten Teilmesssignals S2T, $T_{\alpha i}$ die $\alpha$-Zeitabschnittsdauer eines erkannten $\alpha$-Zeitabschnittes, in dem $\alpha$-Wellen insbesondere dominant sind und/oder vorzugsweise markant hervortreten, und i einen Laufindex bezeichnet.

Bei dem Beispiel gemäß Fig. 12 haben die $\alpha$-Zeitabschnittsdauern die folgenden Sekundenwerte $T_{\alpha 1} = 1{,}1s$, $T_{\alpha 2} = 1{,}0s$, $T_{\alpha 3} = 1{,}2s$ und $T_{\alpha 4} = 1{,}2s$ und die Gesamtzeitdauer des untersuchten zweiten Teilmesssignals S2T hat einen Wert von T = 7s. Die Gleichung (4) liefert einen Wert von 0,64, der größer als der Schwellwert von 0,2 ist, so dass die Steuer- und Auswerteeinheit 3 in diesem Fall keine Insomnie feststellt.

Bei dem Beispiel gemäß Fig. 13 hat die Gesamtzeitdauer des untersuchten zweiten Teilmesssignals S2T einen Wert von T = 8s. Hier gibt es aber keine erkannten $\alpha$-Zeitabschnitte, so dass die Summe aller $\alpha$-Zeitabschnittsdauern $T_{\alpha i}$ den Wert Null ergibt. Die Gleichung (4) liefert demnach ebenfalls einen Wert von Null der kleiner als der Schwellwert von 0,2 ist, so dass die Steuer- und Auswerteeinheit 3 in diesem Fall auf Vorliegen von Insomnie entscheidet.

Insgesamt bietet die Insomnie-Bestimmungs-Vorrichtung 1 erstmals die Möglichkeit einer objektiven Bestimmung von Insomnie. Hierbei kann die Insomnie-Bestimmungs-Vorrichtung 1 vorteilhafterweise vom Patienten 8 selbst bedient werden und vor allem auch in seiner häuslichen Umgebung angewendet werden, was Fehldiagnosen aufgrund einer ungewohnten Schlafumgebung ausschließt und für den Patienten 8 erheblich bequemer ist.

**Patentansprüche**

1. Vorrichtung zur Bestimmung einer bei einem Patienten (8) vorliegenden Insomnie aufweisend

   a) eine Sensoreinheit (2) mindestens zur Erfassung eines EEG-Messsignals eines Gehirnstroms des Patienten (8), und
   b) eine Steuer- und Auswerteeinheit (3) zur Auswertung des erfassten EEG-Messsignals,
   **dadurch gekennzeichnet, dass**
   c) die Steuer- und Auswerteeinheit (3) dazu ausgelegt ist,

      c1) eine objektive Erfassungsroutine durchzuführen, und dabei

         c11) dem Patienten (8) für die folgende Erfassung des EEG-Messsignals eine Vorgabe zur Augenöffnung für die Dauer einer Augenöffnungszeitspanne und zur Augenschließung für die Dauer einer Augenschließungszeitspanne zu machen, und
         c12) danach die Erfassung des EEG-Messsignals während mindestens einer Augenöffnungszeitspanne und während mindestens einer Augenschließungszeitspanne zu veranlassen,

      c2) eine Auswertung des so erfassten EEG-Messsignals durchzuführen, und dabei

         c21) aus dem EEG-Messsignal mindestens ein erstes Teilmesssignal (S1T), das innerhalb der mindestens einen Augenöffnungszeitspanne liegt, und mindestens ein zweites Teilmesssignal (S2T), das innerhalb der mindestens einen Augenschließungszeitspanne liegt, zu extrahieren, und
         c22) auf das Vorliegen von Insomnie zu entscheiden, wenn das innerhalb der mindestens einen Augenschließungszeitspanne liegende mindestens eine zweite Teilmesssignal (S2T) einen geringeren Anteil an $\alpha$-Wellen aufweist als bei einem gesunden Menschen.

2. Vorrichtung nach Anspruch 1, **dadurch gekennzeichnet, dass** die Steuer- und Auswerteeinheit (3) dazu ausgelegt ist, das mindestens eine zweite Teilmesssignal (S2T) auf das Vorliegen von $\alpha$-Zeitabschnitten, in denen $\alpha$-Wellen

hervortreten, zu prüfen, und für jeden erkannten $\alpha$-Zeitabschnitt eine $\alpha$-Zeitabschnittsdauer ($T_{\alpha 1}$, $T_{\alpha 2}$, $T_{\alpha 3}$, $T_{\alpha 4}$) zu ermitteln, und auf das Vorliegen von Insomnie mittleren bis schweren Grades zu entscheiden, wenn die Summe aller $\alpha$-Zeitabschnittsdauern ($T_{\alpha 1}$, $T_{\alpha 2}$, $T_{\alpha 3}$, $T_{\alpha 4}$) bezogen auf eine Gesamtzeitdauer (T) des mindestens einen zweiten Teilmesssignals (S2T) kleiner als 20 % ist.

3. Vorrichtung nach Anspruch 1 oder 2, **dadurch gekennzeichnet, dass** die Steuer- und Auswerteeinheit (3) dazu ausgelegt ist, einen Vergleich zwischen einem ersten Frequenzgehalt des innerhalb der mindestens einen Augenöffnungszeitspanne liegenden mindestens einen ersten Teilmesssignals (S1T) und einem zweiten Frequenzgehalt des innerhalb der mindestens einen Augenschließungszeitspanne liegenden mindestens einen zweiten Teilmesssignals (S2T) durchzuführen, und für den Vergleich

a) aus dem ersten Frequenzgehalt des innerhalb der mindestens einen Augenöffnungszeitspanne liegenden mindestens einen ersten Teilmesssignals (S1T) eine erste $\alpha$-Kenngröße ($\alpha 1$) für ein $\alpha$-Frequenzintervall und eine erste $\beta$-Kenngröße ($\beta 1$) für ein $\beta$-Frequenzintervall sowie ein erstes $\alpha/\beta$-Verhältnis als Quotient der ersten $\alpha$-Kenngröße ($\alpha 1$) zu der ersten $\beta$-Kenngröße ($\beta 1$) zu bestimmen,
b) aus dem zweiten Frequenzgehalt des innerhalb der mindestens einen Augenschließungszeitspanne liegenden mindestens einen zweiten Teilmesssignals (S2T) eine zweite $\alpha$-Kenngröße ($\alpha 2$) für das $\alpha$-Frequenzintervall und eine zweite $\beta$-Kenngröße ($\beta 2$) für das $\beta$-Frequenzintervall sowie ein zweites $\alpha/\beta$-Verhältnis als Quotient der zweiten $\alpha$-Kenngröße ($\alpha 2$) zu der zweiten $\beta$-Kenngröße ($\beta 2$) zu bestimmen, und
c) eine relative Änderung zwischen dem ersten und dem zweiten $\alpha/\beta$-Verhältnis zu bestimmen.

4. Vorrichtung nach Anspruch 3, **dadurch gekennzeichnet, dass** die Steuer- und Auswerteeinheit (3) dazu ausgelegt ist,

a) die relative Änderung zwischen dem ersten und dem zweiten $\alpha/\beta$-Verhältnis als eine auf das erste $\alpha/\beta$-Verhältnis bezogene Differenz zwischen dem ersten $\alpha/\beta$-Verhältnis als Minuend und dem zweiten $\alpha/\beta$-Verhältnis als Subtrahend zu bestimmen, und
b) auf das Vorliegen von Insomnie mittleren bis schweren Grades zu entscheiden, wenn die so bestimmte relative Änderung zwischen dem ersten und dem zweiten $\alpha/\beta$-Verhältnis größer als ein Insomnie-Grenzwert (27) ist.

5. Vorrichtung nach Anspruch 3, **dadurch gekennzeichnet, dass** die Steuer- und Auswerteeinheit (3) dazu ausgelegt ist, einen Insomnie-Grad IG gemäß der Gleichung:

$$IG = e^{\left(\frac{\Delta\alpha\beta + c1}{c2}\right)}$$

mit

$$\Delta\alpha\beta = \frac{\left(\frac{\alpha 1}{\beta 1} - \frac{\alpha 2}{\beta 2}\right)}{\frac{\alpha 1}{\beta 1}}$$

zu bestimmen, wobei c1 eine erste Konstante, c2 eine zweite Konstante, $\Delta\alpha\beta$ die relative Änderung zwischen dem ersten und dem zweiten $\alpha/\beta$-Verhältnis, $\alpha 1$ die erste $\alpha$-Kenngröße während der Augenöffnungszeitspanne, $\beta 1$ die erste $\beta$-Kenngröße während der Augenöffnungszeitspanne, $\alpha 2$ die zweite $\alpha$-Kenngröße während der Augenschlie-ßungszeitspanne und $\beta 2$ die zweite $\beta$-Kenngröße während der Augenschließungszeitspanne bezeichnet.

6. Vorrichtung nach einem der vorhergehenden Ansprüche, **dadurch gekennzeichnet, dass** die Steuer- und Auswerteeinheit (3) dazu ausgelegt ist, die objektive Erfassungsroutine vor einem Einschlafen des Patienten (8) durchzuführen.

7. Vorrichtung nach einem der vorhergehenden Ansprüche, **dadurch gekennzeichnet, dass** die Vorrichtung mindestens eine portable Teilkomponente (14, 15) aufweist.

8. Vorrichtung nach einem der vorhergehenden Ansprüche, **dadurch gekennzeichnet, dass** die Vorrichtung (1) als eine vom Patienten (8) selbst bedienbare Vorrichtung (1) ausgeführt ist.

9. Vorrichtung nach einem der vorhergehenden Ansprüche, **dadurch gekennzeichnet, dass** die Vorrichtung (1) als eine vom Patienten (8) in seiner häuslichen Umgebung bedienbare Vorrichtung ausgeführt (1) ist.

10. Vorrichtung nach einem der vorhergehenden Ansprüche, **dadurch gekennzeichnet, dass** die Steuer- und Auswerteeinheit (3) dazu ausgelegt ist, vor der Extraktion des mindestens einen ersten Teilmesssignals (S1T) und des mindestens einen zweiten Teilmesssignals (S2T) eine Artefaktbereinigung des EEG-Messsignals durchzuführen.

11. Vorrichtung nach einem der vorhergehenden Ansprüche, **dadurch gekennzeichnet, dass** die Steuer- und Auswerteeinheit (3) dazu ausgelegt ist, für die mindestens eine Augenöffnungszeitspanne und die mindestens eine Augenschließungszeitspanne jeweils eine Zeitdauer von mindestens 2 Sekunden vorzusehen.

12. Vorrichtung nach einem der vorhergehenden Ansprüche, **dadurch gekennzeichnet, dass** die Steuer- und Auswerteeinheit (3) dazu ausgelegt ist, während der objektiven Erfassungsroutine mindestens zwei Augenöffnungszeitspannen und mindestens zwei Augenschließungszeitspannen vorzusehen, wobei sich die Augenöffnungszeitspannen und die Augenschließungszeitspannen jeweils abwechseln und insbesondere jeweils unmittelbar aufeinander folgen.

13. Vorrichtung nach einem der vorhergehenden Ansprüche, **dadurch gekennzeichnet, dass** die Steuer- und Auswerteeinheit (3) dazu ausgelegt ist, dem Patienten (8) während der Durchführung der objektiven Erfassungsroutine anzuzeigen, wann die mindestens eine Augenöffnungszeitspanne und die mindestens eine Augenschließungszeitspanne jeweils beginnt und endet.

14. Vorrichtung nach einem der vorhergehenden Ansprüche, **dadurch gekennzeichnet, dass** sie mehrkomponentig ausgeführt ist.

15. Vorrichtung nach einem der vorhergehenden Ansprüche, **dadurch gekennzeichnet, dass** die Steuer- und Auswerteeinheit (3) dazu ausgelegt ist, eine subjektive Erfassungsroutine durchzuführen, und dabei mit dem Patienten (8) eine Befragung zu einer subjektiven Schlafwahrnehmung vorzunehmen und insbesondere anhand der Befragungsergebnisse eine weitere Insomnie-Bestimmung vorzunehmen.

Fig. 1

Fig. 2

Fig. 3

Fig. 4

Fig. 5

Fig. 6

Fig. 7

Fig. 8

Fig. 9

Fig. 10

Fig. 11

EP 4 706 508 A1

Fig. 12

Fig. 13

Europäisches
Patentamt

European
Patent Office

Office européen
des brevets

## EUROPÄISCHER RECHERCHENBERICHT

**Nummer der Anmeldung**

EP 25 19 5708

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | COLOMBO MICHELE A. ET AL: "Wake High-Density Electroencephalographic Spatiospectral Signatures of Insomnia", SLEEP, Bd. 39, Nr. 5, 1. Mai 2016 (2016-05-01), Seiten 1015-1027, XP093351246, US ISSN: 0161-8105, DOI: 10.5665/sleep.5744 * Methods: S. 1016-1018; Abbildungen 1,2 * ----- | 1-15 | INV. A61B5/00 |

RECHERCHIERTE
SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| Berlin | 19. Januar 2026 | Trachterna, Morten |

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102024208368
- DE 202022106837 U1